# EUROPÄISCHE PATENTSCHRIFT

(11) **EP 2 768 403 B1**
(45) Veröffentlichungstag und Bekanntmachung des Hinweises auf die Patenterteilung: **08.07.2015**
(21) Anmeldenummer: 12772973.9
(22) Anmeldetag: 18.10.2012
(51) Int. Cl.: A61B 17/32, A61B 17/14

(54) **SONOTRODE**
SONOTRODE
SONOTRODE

(30) Priorität: 19.10.2011 DE 102011084792
(43) Veröffentlichungstag der Anmeldung: 27.08.2014
(73) Patentinhaber: Söring GmbH, 25451 Quickborn (DE)
(72) Erfinder: RAD, Abtin Jamshidi, 22115 Hamburg (DE)
(74) Vertreter: Glawe, Delfs, Moll
(86) Internationale Anmeldenummer: PCT/EP2012/070636
(87) Internationale Veröffentlichungsnummer: WO 2013/057179

(56) Entgegenhaltungen:
- EP-A1- 0 456 470
- GB-A- 2 107 641
- US-A- 5 695 510
- US-A1- 2006 123 959
- US-A1- 2007 233 131
- US-A1- 2010 168 741

## Beschreibung

Die Erfindung betrifft eine Sonotrode für ein ultraschallchirurgisches Instrument. Die Sonotrode hat einen Instrumentenkopf, der mit einer sich in Längsrichtung erstreckenden Schneideinrichtung ausgestattet ist.

Ultraschallchirurgische Instrumente werden bei chirurgischen Operationen dazu verwendet, um Gewebe eines Patienten zu durchtrennen. Mit einem Ultraschallwandler wird eine hochfrequente mechanische Schwingung erzeugt. Die Sonotrode ist an den Ultraschallwandler angeschlossen und wird durch den Ultraschallwandler in Schwingung versetzt. Wenn die Schneideinrichtung der Sonotrode mit dem Gewebe in Kontakt gebracht wird, wird das Gewebe durch die hochfrequente Schwingung durchtrennt.

Dokument US 2010/168741 A1 offenbart eine Sonotrode entsprechend dem Oberbegriff des Anspruchs 1.

Zum Schneiden von Weichgewebe werden ultraschallchirurgische Instrumente dieser Art seit langem mit Erfolg eingesetzt. Wünschenswert ist es, die Vorteile der ultraschallchirurgischen Instrumente auch beim Bearbeiten und Durchtrennen von Knochen zu nutzen. Tatsächlich sind die hochfrequenten Schwingungen des Instrumentenkopfs geeignet, die Struktur des Knochenmaterials aufzulösen. Allerdings hindert das aufgelöste Knochenmaterial den Instrumentenkopf daran, weiter in den Knochen einzudringen und einen tieferen Schnitt zu erzeugen. Zum Bearbeiten und Durchtrennen von Knochenmaterial sind die bekannten Sonotroden deswegen weniger geeignet.

Der Erfindung liegt die Aufgabe zu Grunde, eine Sonotrode vorzustellen, die eine gute Eignung zum Bearbeiten und Durchtrennen von Knochenmaterial hat. Ausgehend vom eingangs genannten Stand der Technik wird die Aufgabe gelöst mit den Merkmalen des Anspruchs 1. Vorteilhafte Ausführungsformen finden sich in den Unteransprüchen.

Erfindungsgemäß ist der Instrumentenkopf mit einer Mehrzahl von Durchbrechungen versehen, die sich in Querrichtung durch den Instrumentenkopf hindurch erstrecken. Die Durchbrechungen haben im Querschnitt eine längliche Form, wobei die größte Querschnittsausdehnung der Durchbrechung und die Längsrichtung des Instrumentenkopfs einen Winkel zwischen sich einschließen, der zwischen 30° und 80° liegt.

Mit den erfindungsgemäßen Durchbrechungen werden die mechanischen Eigenschaften des Instrumentenkopfs gezielt beeinflusst. Gegenüber niederfrequenten Kräften hat der Instrumentenkopf eine ähnliche Steifigkeit wie klassische Sonotroden. Der Chirurg kann also wie gewohnt Druck auf den Arbeitsbereich ausüben oder eine manuelle Sägebewegung durchführen. Gegenüber den hochfrequenten Schwingungen des Ultraschallwandlers hingegen wird dem Instrumentenkopf mit den Durchbrechungen eine elastische In-Sich-Beweglichkeit verliehen. Indem die Durchbrechungen quer zur Längsrichtung des Instrumentenkopfs ausgerichtet sind, vollführt der Instrumentenkopf eine In-Sich-Bewegung, die der durch den Ultraschallwandler angetriebenen hochfrequenten Schwingung überlagert ist. Diese kombinierte Bewegung führt dazu, dass einerseits ständig neues Knochenmaterial in seiner Struktur aufgelöst wird und andererseits das aufgelöste Knochenmaterial aus dem Arbeitsbereich abtransportiert wird. Durch den Abtransport des aufgelösten Knochenmaterials entsteht Platz, in den der Instrumentenkopf eindringen kann, um weiteres Knochenmaterial aufzulösen. Der Instrumentenkopf kann auf diese Weise immer weiter in den Knochen eindringen und eine Schnittfuge erzeugen. Außerdem wird durch den schnellen Abtransport des aufgelösten Knochenmaterials der Temperaturanstieg in Grenzen gehalten.

Im Querschnitt (also in einer senkrecht zur Mittelachse der Durchbrechung ausgerichteten Ebene) betrachtet haben die Durchbrechungen eine längliche Form. Dabei ist die größte Querschnittsausdehnung vorzugsweise mindestens doppelt so groß, weiter vorzugsweise mindestens dreimal so groß wie kleinste Querschnittsausdehnung. Die größte Querschnittsausdehnung ist vorzugsweise so lang, dass sie mindestens 50% der Höhe des Instrumentenkopfs überdecken kann.

Die größte Querschnittsausdehnung der Durchbrechung schließt mit der Längsrichtung des Instrumentenkopfs einen Winkel ein. Dies hat den Vorteil, dass die Kräfte im Instrumentenkopf umgelenkt werden und die elastische Bewegung dadurch eine andere Richtung erhält als die hochfrequente Schwingung. Das aufgelöste Knochenmaterial wird dann besonders wirksam abtransportiert. Der Winkel zwischen der größten Querschnittsausdehnung der Durchbrechung und der Längsrichtung des Instrumentenkopfs liegt zwischen 30° und 80°, vorzugsweise zwischen 50° und 70°.

Die Durchbrechungen sollten so angeordnet sein, dass die elastische In-Sich-Beweglichkeit des Instrumentenkopfs ausgeprägt ist. Dazu ist es von Vorteil, wenn die Mittelachsen der Durchbrechungen parallel zueinander ausgerichtet sind. Außerdem können die Durchbrechungen in Längsrichtung des Instrumentenkopfs hintereinander angeordnet sein. Im Fall, dass die Mittelachsen der Durchbrechungen parallel zueinander angeordnet sind, bedeutet dies, dass die Mittelachsen in einer Ebene liegen, die durch die Längsrichtung und die Querrichtung des Instrumentenkopfs aufgespannt ist.

Das schneidflächenseitige Ende der im Querschnitt länglichen Durchbrechung liegt vorzugsweise näher dem hinteren (proximalen) Ende des Instrumentkopfs als das gegenüberliegende Ende. Die elastische Beweglichkeit des Instrumentenkopfs führt dann zu einer Nickbewegung, durch die das aufgelöste Knochenmaterial besonders wirksam abtransportiert wird.

Für die elastische Beweglichkeit des Instrumentenkopfs ist es von Vorteil, wenn die Durchbrechungen nah beieinander liegen. Beispielsweise können die Durchbrechungen so angeordnet sein, dass der Abstand der Mittelachsen zweier benachbarter Durchbrechungen höchstens doppelt so groß ist wie die Querschnittsausdehnung der Durchbrechungen in der betreffenden Richtung. Wenn die Durchbrechungen im Querschnitt länglich sind, bleibt zwischen zwei Durchbrechungen jeweils ein Materialsteg. Dieser Materialsteg ist vorzugsweise mindestens doppelt so lang wie breit. Die mit den erfindungsgemäßen Durchbrechungen erzielte elastische Beweglichkeit des Instrumentenkopfs hat zudem den Vorteil, dass Quietschgeräusche im Vergleich mit in sich starren Sonotroden vermindert sind.

Die Schneideinrichtung des Instrumentenkopfs hat vorzugsweise eine flächenhafte Ausdehnung. Bei einer solchen Schneidfläche ist die Gefahr einer unbeabsichtigten Verletzung des Gewebes geringer als bei einer scharfen Schneide. Die Schneidfläche ist in einer vorteilhaften Ausführungsform im Wesentlichen parallel zu einer von den Mittelachsen der Durchbrechungen aufgespannten Fläche ausgerichtet. Die Amplitude und Frequenz der hochfrequenten Schwingung werden vorzugsweise so eingestellt, dass die Schneidwirkung bei einfacher Berührung des Knochens nur gering ist. Erst durch Überlagerung der hochfrequenten Schwingung mit einer vom Chirurgen durchgeführten manuellen Sägebewegung wird die Schneidwirkung maximal.

Die Schneidfläche kann mit einer Mehrzahl von Ausnehmungen versehen sein, die sich in Querrichtung des Instrumentenkopfs erstrecken. In Längsrichtung des Instrumentenkopfs kann eine Mehrzahl dieser Ausnehmungen hintereinander angeordnet sein. Wenn die hochfrequente Schwingung der Sonotrode eine Bewegung in Längsrichtung des Instrumentenkopfs ist, sind die Ausnehmungen quer zur Bewegungsrichtung ausgerichtet. Im Querschnitt können die Ausnehmungen beispielsweise annähernd halbkreisförmig sein. Mit der durch die Ausnehmungen gebildeten Struktur kann das Knochenmaterial wirksam bearbeitet werden.

In einer vorteilhaften Ausführungsform verjüngen sich die Ausnehmungen vom Rand der Schneidfläche in Richtung des Zentrums, so dass die Ausnehmungen eine sanduhrähnliche Form haben. In Längsrichtung des Instrumentenkopfs betrachtet ist der Abstand zweier benachbarter Ausnehmungen dann im Zentrum größer als am Rand der Schneidfläche. Im Zentrum der Schneidfläche gibt es also einen rautenähnlichen Flächenabschnitt zwischen zwei Ausnehmungen, der vorzugsweise im Wesentlichen eben ist. Dieser Flächenabschnitt wirkt als Kontaktfläche, die flächig auf dem zu bearbeitenden Knochen aufliegt. Am Rand der Schneidfläche grenzen die Ausnehmungen - in Längsrichtung des Instrumentenkopfs betrachtet - vorzugsweise direkt aneinander.

Diese Gestaltung der Ausnehmungen hat den Vorteil, dass der Knochen durch die Kanten am Übergang zwischen den Kontaktflächen und den Ausnehmungen wirksam bearbeitet werden kann. Dabei wird das aufgelöste Knochenmaterial durch die schräg ausgerichteten Kanten nach außen geleitet. Andererseits wird Weichgewebe, mit dem die Sonotrode versehentlich in Berührung kommt, nicht geschädigt. Bei geeignet ausgewählter Frequenz und Amplitude der hochfrequenten Schwingung wird nämlich das Weichgewebe durch die flächig aufliegenden Kontaktflächen selbst in Schwingung versetzt, anstatt durchtrennt zu werden. Wenn die Sonotrode auf Weichgewebe aufliegt, verändert sich auch das Schwingungsverhalten des Instruments, so dass der Chirurg eine unmittelbare Rückmeldung erhält, wenn er nicht mehr am Knochen arbeitet. Mit dem erfindungsgemäßen Instrument kann deswegen auch in unmittelbarer Nähe zu beispielsweise Nerven oder Blutgefäßen gearbeitet werden. Diese Gestaltung der Ausnehmungen hat eigenständigen erfinderischen Gehalt, auch ohne dass der Instrumentenkopf mit Durchbrechungen versehen ist.

Die Sonotrode umfasst regelmäßig einen mit dem Instrumentenkopf verbundenen Schaft. Über das andere Ende des Schafts kann eine Verbindung zu dem Ultraschallwandler hergestellt werden. Der Schaft ist vorzugsweise in Längsrichtung des Instrumentenkopfs ausgerichtet. Der Schaft bildet das proximale Ende der Sonotrode, das distale Ende liegt gegenüber. In Bezug auf den Instrumentenkopf werden die Begriffe proximal und distal entsprechend verwendet.

Die Ausdehnung des Instrumentenkopfs in Längsrichtung kann zwischen 5 mm und 15 mm, vorzugsweise zwischen 8 mm und 10 mm liegen. Die Ausdehnung des Instrumentenkopfs in Querrichtung kann beispielsweise zwischen 1 mm und 5 mm liegen. Regelmäßig ist die Ausdehnung in Längsrichtung größer als die Ausdehnung in Querrichtung. Die zur Längsrichtung und Querrichtung senkrechte Höhenausdehnung kann ebenfalls zwischen 1 mm und 5 mm liegen.

Die Erfindung betrifft außerdem ein chirurgisches Instrument mit einem Ultraschallwandler und einer an den Ultraschallwandler angeschlossenen erfindungsgemäßen Sonotrode. Der Ultraschallwandler kann ein Piezoelement umfassen, mit dem eine hochfrequente Wechselspannung in eine entsprechende mechanische Schwingung umgesetzt wird. Die Frequenz der Schwingung kann beispielsweise zwischen 20 kHz und 40 kHz liegen.

Das ultraschallchirurgische Instrument ist regelmäßig mit einer Leitung ausgestattet, über die dem Operationsfeld eine Spülflüssigkeit zugeführt werden kann. Durch die Bewegung der Sonotrode bilden sich in der Umgebung des Instrumentenkopfs Spülflüssigkeitswirbel, die auch in die Durchbrechungen eintreten und dadurch eine wirksame Kühlung des Instrumentenkopfs bewirken.

Die Erfindung wird nachfolgend unter Bezugnahme auf die beigefügten Zeichnungen anhand einer vorteilhaften Ausführungsform beispielhaft beschrieben. Es zeigen:
- Fig. 1:: eine Seitenansicht eines erfindungsgemäßen ultraschallchirurgischen Instruments;
- Fig. 2:: eine Seitenansicht einer erfindungsgemäßen Sonotrode;
- Fig. 3:: den Instrumentenkopf der Sonotrode aus Fig. 2 in vergrößerter Darstellung; und
- Fig. 4:: eine Ansicht von unten auf den Instrumentenkopf aus Fig. 2.

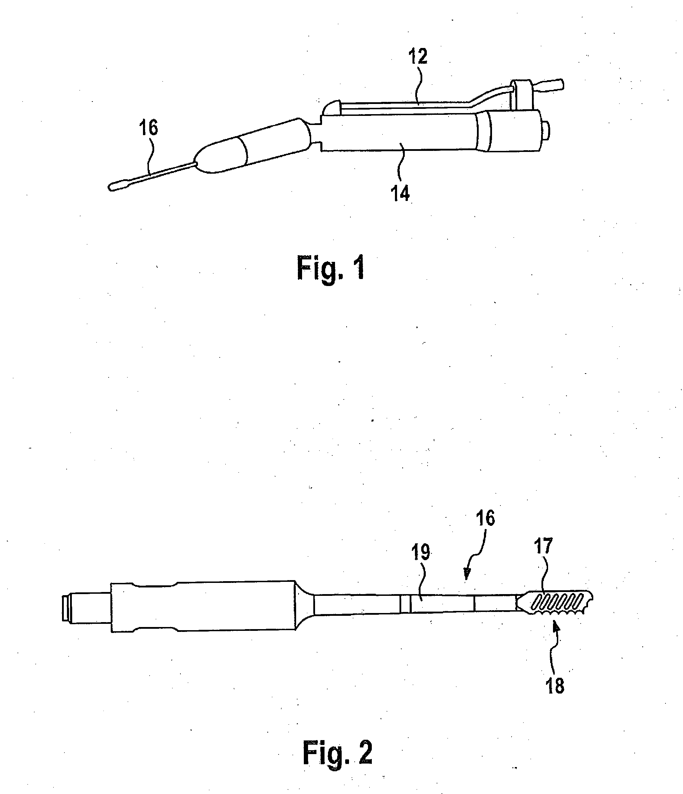

Ein ultraschallchirurgisches Instrument in Fig. 1 umfasst an seinem hinteren Ende einen Handgriff 14, über den der Chirurg das Instrument führen kann. Im Inneren des Instruments ist ein in Fig. 1 nicht sichtbarer Ultraschallwandler angeordnet, der als Eingangssignal ein elektrisches Wechselspannungssignal von einem in Fig. 1 ebenfalls nicht dargestellten Signalgenerator erhält. Die Frequenz der Wechselspannung kann beispielsweise zwischen 20 kHz und 40 kHz liegen. Der Ultraschallwandler umfasst ein Piezoelement, mit dem das elektrische Signal in eine mechanische Schwingung umgewandelt wird, die in Längsrichtung des Instruments ausgerichtet ist. Die mechanische Schwingung wird auf eine Sonotrode 16 übertragen, die einen Schaft 19 mit einem Instrumentenkopf 17 am vorderen Ende umfasst. Die in Fig. 1 unten dargestellte Fläche des Instrumentenkopfs 17 dient als Schneidfläche 18. Wird der in Schwingung stehende Instrumentenkopf 17 mit der in Schwingung stehenden Schneidfläche 18 an einem Knochen herangeführt, wird das Knochenmaterial durchtrennt. Das ultraschallchirurgische Instrument umfasst außerdem eine in Fig. 1 sichtbare Leitung 12, um dem Operationsfeld eine Spülflüssigkeit zuführen zu können.

Der Instrumentenkopf 17 der Sonotrode 16 aus Fig. 1 ist in Fig. 2 in einer vergrößerten Darstellung gezeigt. Danach ist der Instrumentenkopf 17 mit sechs Durchbrechungen 20 versehen, die sich in Querrichtung (in Fig. 2 senkrecht zur Bildebene) durch den Instrumentenkopf 17 hindurch erstrecken. Bei den Durchbrechungen 20 handelt es sind um Kanäle, die den Instrumentenkopf vollständig durchqueren. Im Querschnitt betrachtet (in Fig. 2 in der Bildebene) haben die Durchbrechungen 20 eine längliche Form, wobei die größte Querschnittsausdehnung schräg von links unten nach rechts oben verläuft. Mit der Längsrichtung der Sonotrode schließt die größte Querschnittsausdehnung einen Winkel α von etwa 60° ein.

Die Mittelachsen der Durchbrechungen 20 sind parallel zueinander angeordnet und liegen alle in einer Ebene, die durch die Längsachse der Sonotrode 16 und die Querrichtung aufgespannt wird. Der Abstand der Mittelachsen ist derart, dass die jeweils zwischen zwei Durchbrechungen 20 bleibenden Stege 21 dünn sind verglichen mit der größten Querschnittsausdehnung der Durchbrechungen 20.

Aufgrund der Durchbrechungen 20 ist der Instrumentenkopf 17 in sich elastisch, so dass er gegenüber den hochfrequenten Schwingungen der Sonotrode 16 wie ein Federelement wirkt. Vibriert die Sonotrode 16 in Längsrichtung, so kommt es in dem Instrumentenkopf 17 zu einer elastischen In-Sich-Bewegung in Form einer Nickbewegung. Die Nickbewegung findet im Wesentlichen in der Bildebene der Fig. 1 statt.

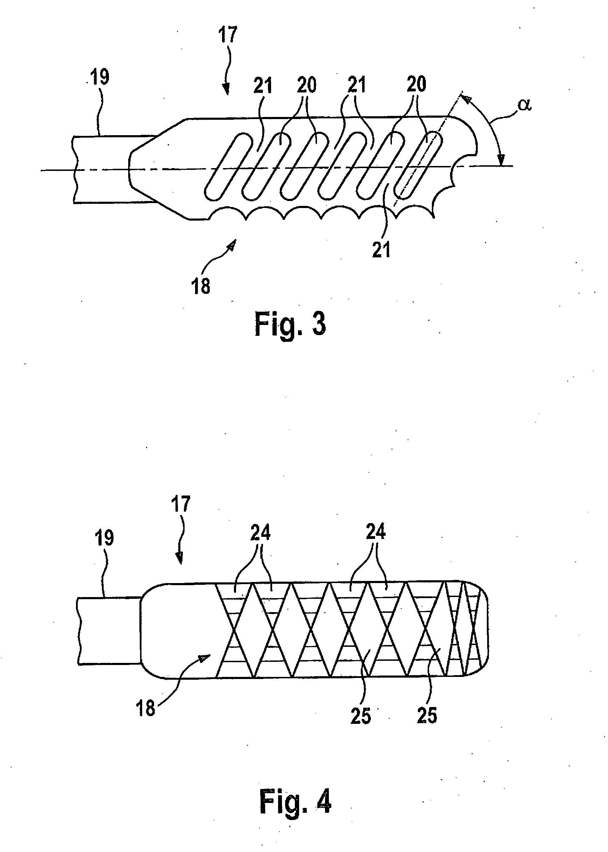

In Fig. 3 ist eine Ansicht von unten auf den Instrumentenkopf 17 aus Fig. 2 gezeigt und damit eine Draufsicht auf die Schneidfläche 18 des Instrumentenkopfs 17. In der Schneidfläche 18 ist eine Mehrzahl von Ausnehmungen 24 ausgebildet. Die Ausnehmungen 24 verjüngen sich von beiden Seiten vom Rand zum Zentrum hin, so dass sie eine sanduhrähnliche Form haben. Im Querschnitt sind die Ausnehmungen 24 etwa halbkreisförmig. Am Rand grenzen jeweils zwei benachbarte Ausnehmungen direkt aneinander. Im Zentrum haben die Ausnehmungen 24 einen Abstand zueinander, der von einer ebenen Kontaktfläche 25 ausgefüllt wird. In Fig. 3 haben die Kontaktflächen 25 die Form eines Rhombus.

Bei der Verwendung des erfindungsgemäßen ultraschallchirurgischen Instruments setzt der Chirurg zunächst den Ultraschallwandler in Betrieb, so dass die Sonotrode 16 in eine Schwingung in Längsrichtung versetzt wird. Der Chirurg bewegt dann das Instrument so, dass der Instrumentenkopf 17 mit der Schneidfläche 18 eine manuelle Sägebewegung auf dem zu durchtrennenden Knochen vollführt. Durch die Überlagerung der manuellen Sägebewegung mit der hochfrequenten Schwingung wird das Knochenmaterial durchtrennt. Dabei reicht es aus, wenn ein leichter Druck von etwa 100 g auf das Instrument ausgeübt wird. Die Schneidwirkung ergibt sich insbesondere durch die Kanten zwischen den Kontaktflächen 25 und den Ausnehmungen 24. Die Struktur des Knochenmaterials wird aufgelöst, so dass der Instrumentenkopf 17 in das Knochenmaterial eindringen kann. Es entsteht ein Schnitt, dessen Breite der Breite des Instrumentenkopfs entspricht.

Durch die Nickbewegung, die der Instrumentenkopf 17 in sich vollführt, während die Sonotrode 16 in Schwingung ist, wird das in seiner Struktur aufgelöste Knochenmaterial aus dem Arbeitsbereich abgeführt. Der Instrumentenkopf 17 ist also nicht daran gehindert, tiefer in das Knochenmaterial einzudringen. Der Abtransport des aufgelösten Knochenmaterials wird außerdem durch die Gestaltung der Ausnehmungen 24 gefördert, die schräg nach außen weisende Flächen umfasst.

Kommt der Instrumentenkopf mit Weichgewebe anstatt mit Knochenmaterial in Berührung, ist die Schneidwirkung nur gering. Durch die großen Kontaktflächen 25 zwischen den Ausnehmungen 24 liegt die Schneidfläche 18 im Wesentlichen flächig auf dem Weichgewebe auf. Das Weichgewebe wird deswegen nicht durchtrennt, sondern lediglich in Schwingung versetzt. Das erfindungsgemäße Instrument bietet also einen Schutz dagegen, dass durch unsauberes Führen des Instruments versehentlich Weichgewebe anstatt des Knochens durchtrennt wird.

## Patentansprüche

1. Sonotrode für ein ultraschallchirurgisches Instrument, mit einem Instrumentenkopf (17), der mit einer sich in Längsrichtung erstreckenden Schneideinrichtung (18) ausgestattet ist, wobei der Instrumentenkopf (17) mit einer Mehrzahl von Durchbrechungen (20) versehen ist, die sich in Querrichtung durch den Instrumentenkopf (17) hindurch erstrecken und wobei die Durchbrechungen (20) im Querschnitt eine längliche Form haben, **dadurch gekennzeichnet, dass** die größte Querschnittsausdehnung der Durchbrechungen (20) und die Längsrichtung des Instrumentenkopfs (17) einen Winkel zwischen sich einschließen, der zwischen 30° und 80° liegt.

2. Sonotrode nach Anspruch 1, **dadurch gekennzeichnet, dass** die Mittelachsen der Durchbrechungen (20) parallel zueinander angeordnet sind.

3. Sonotrode nach Anspruch 1 oder 2, **dadurch gekennzeichnet, dass** die Durchbrechungen (20) in Längsrichtung des Instrumentenkopfs (17) hintereinander angeordnet sind.

4. Sonotrode nach einem der Ansprüche 1 bis 3, **dadurch gekennzeichnet, dass** der Winkel zwischen der größten Querschnittsausdehnung der Durchbrechung (20) und der Längsrichtung des Instrumentenkopfs (17) zwischen 50° und 70° liegt.

5. Sonotrode nach einem der Ansprüche 1 bis 4, **dadurch gekennzeichnet, dass** das der Schneideinrichtung zugewandte Ende der Durchbrechung (20) näher am proximalen Ende des Instrumentenkopfs (17) liegt als das von der Schneideinrichtung entfernte Ende der Durchbrechung (20).

6. Sonotrode nach einem der Ansprüche 1 bis 5, **dadurch gekennzeichnet, dass** die Schneideinrichtung als Schneidfläche (18) ausgebildet ist.

7. Sonotrode nach Anspruch 6, **dadurch gekennzeichnet, dass** die Schneidfläche (18) im Wesentlichen parallel zu einer von den Mittelachsen der Durchbrechungen (20) aufgespannten Fläche ausgerichtet ist.

8. Sonotrode nach einem der Ansprüche 1 bis 7, **dadurch gekennzeichnet, dass** die Schneidfläche mit einer Mehrzahl von Ausnehmungen (24) versehen ist, die sich in Querrichtung des Instrumentenkopfs (17) erstrecken.

9. Sonotrode nach Anspruch 8, **dadurch gekennzeichnet, dass**.die Ausnehmungen (24) sich vom Rand des Instrumentenkopfs (17) in Richtung des Zentrums verjüngen.

10. Chirurgisches Instrument mit einem Ultraschallwandler und einer an den Ultraschallwandler angeschlossenen Sonotrode, **dadurch gekennzeichnet, dass** die Sonotrode nach einem,der Ansprüche 1 bis 9 ausgebildet ist.

## Claims

1. Sonotrode for an ultrasonic surgical instrument, comprising an instrument head (17) which is equipped with a cutting apparatus (18) that extends in the longitudinal direction, wherein the instrument head (17) is provided with a plurality of through-holes (20), which extend through the instrument head (17) in the transverse direction and wherein in terms of the cross section, the through-holes (20) have an elongate form, **characterized in that** the largest cross-sectional extent of the through-holes (20) and the longitudinal direction of the instrument head (17) include an angle therebetween which lies between 30° and 80°.

2. Sonotrode according to Claim 1, **characterized in that** the central axes of the through-holes (20) are arranged parallel to one another.

3. Sonotrode according to Claim 1 or 2, **characterized in that** the through-holes (20) are arranged behind one another in the longitudinal direction of the instrument head (17).

4. Sonotrode according to one of Claims 1 to 3, **characterized in that** the angle between the largest cross-sectional extent of the through-hole (20) and the longitudinal direction of the instrument head (17) is between 50° and 70°.

5. Sonotrode according to one of Claims 1 to 4, **characterized in that** the end of the through-hole (20) facing the cutting apparatus is closer to the proximal end of the instrument head (17) than the end of the through-hole (17) distant from the cutting apparatus.

6. Sonotrode according to one of Claims 1 to 5, **characterized in that** the cutting apparatus is embodied as cutting surface (18).

7. Sonotrode according to Claim 6, **characterized in that** the cutting surface (18) is aligned substantially parallel to a surface spanned by the central axes of the through-holes (20).

8. Sonotrode according to one of Claims 1 to 7, **characterized in that** the cutting surface is provided with a plurality of cut-outs (24), which extend in the transverse direction of the instrument head (17).

9. Sonotrode according to Claim 8, **characterized in that** the cut-outs (24) taper off from the edge of the instrument head (17) in the direction of the centre.

10. Surgical instrument with an ultrasound transducer and a sonotrode connected to the ultrasound transducer, **characterized in that** the sonotrode is embodied according to one of Claims 1 to 9.

## Revendications

1. Sonotrode pour un instrument chirurgical à ultrasons, avec une tête d'instrument (17), qui est munie d'un dispositif de coupe (18) s'étendant dans la direction longitudinale, la tête d'instrument (17) étant munie d'une pluralité d'ouvertures (20) qui s'étendent à travers la tête d'instrument (17) dans la direction transversale et les ouvertures (20) ayant une section transversale de forme oblongue, **caractérisée en ce que** l'extension de section transversale la plus grande des ouvertures (20) et la direction longitudinale de la tête d'instrument (17) forment entre elles un angle entre 30° et 80°.

2. Sonotrode selon la revendication 1, **caractérisée en ce que** les axes centraux des ouvertures (20) sont parallèles entre eux.

3. Sonotrode selon la revendication 1 ou 2, **caractérisée en ce que** les ouvertures (20) sont disposées successivement dans la direction longitudinale de la tête d'instrument (17).

4. Sonotrode selon l'une des revendications 1 à 3, **caractérisée en ce que** l'angle entre l'extension de section transversale la plus grande de l'ouverture (20) et la direction longitudinale de la tête d'instrument (17) est entre 50° et 70°.

5. Sonotrode selon l'une des revendications 1 à 4, **caractérisée en ce que** l'extrémité de l'ouverture (20) orientée vers le dispositif de coupe est plus proche de l'extrémité proximale de la tête d'instrument (17) que l'extrémité de l'ouverture (20) éloignée du dispositif de coupe.

6. Sonotrode selon l'une des revendications 1 à 5, **caractérisée en ce que** le dispositif de coupe est conçu comme une surface de coupe (18).

7. Sonotrode selon la revendication 6, **caractérisée en ce que** la surface de coupe (18) est globalement parallèle à une surface délimitée par les axes centraux des ouvertures (20).

8. Sonotrode selon l'une des revendications 1 à 7, **caractérisée en ce que** la surface de coupe est munie d'une pluralité d'évidements (24) qui s'étendent dans la direction transversale de la tête d'instrument (17).

9. Sonotrode selon la revendication 8, **caractérisée en ce que** les évidements (24) se rétrécissent du bord de la tête d'instrument (17) en direction du centre.

10. Instrument chirurgical avec un convertisseur à ultrasons et une sonotrode branchée au convertisseur à ultrasons, **caractérisé en ce que** la sonotrode est conçue selon l'une des revendications 1 à 9.
